# EUROPEAN PATENT APPLICATION

(11) **EP 4 410 818 A1**
(43) Date of publication of application: **07.08.2024**
(21) Application number: 22876779.4
(22) Date of filing: 27.09.2022
(51) Int. Cl.: C07K 7/08, A61K 38/00, A61P 17/00, A61K 8/64, A61Q 19/00

(54) **PEPTIDE HAVING ANTI-AGING ACTIVITY, AND USE THEREOF**

(30) Priority: 29.09.2021 KR 20210129258
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); LEE, Eung Ji, Anyang-si, Gyeonggi-do 14119 (KR); KANG, Hana, Anyang-si, Gyeonggi-do 14119 (KR); HWANG, Bobyeol, Anyang-si, Gyeonggi-do 14119 (KR)
(74) Representative: Regimbeau
(86) International application number: PCT/KR2022/014392
(87) International publication number: WO 2023/055007

(57) **Abstract**

The present invention relates to a peptide having anti-aging activity and a composition for skin regeneration or suppressing skin aging, comprising the peptide as an active ingredient. The peptide according to the present invention, comprising an amino acid sequence of SEQ ID NO: 1, has anti-aging activity and can thus be used as a raw material for a cosmetic product for skin regeneration or suppressing skin aging, or as a raw material for a pharmaceutical product for preventing, ameliorating, or treating photoaging.

## Description

### TECHNICAL FIELD

The present invention relates to a peptide having anti-aging activity and a composition for skin aging inhibition or skin regeneration containing the peptide as an active ingredient.

### BACKGROUND ART

Skin cells may age due to various causes, and wrinkles may occur due to aging of skin cells. The aging may be classified into chronological aging (endogenous aging), which is natural aging that occurs over time according to a biological process, and photoaging (actinic aging) in which a degenerative change that occurs in an area exposed to sunlight and the chronological aging occur in combination. When expression or activity of various factors related to cell growth is inhibited, natural cell aging may occur, and photoaging of skin cells may occur due to light with a wavelength of 280 nm to 400 nm included in sunlight. In particular, irradiation of ultraviolet rays such as UVB with a wavelength in a range of 280 nm to 320 nm may cause damage to skin or fibers and may make the skin tan, which may darken the skin color. When skin cells are irradiated with UVB, accumulation of ROS and free radicals in the skin cells is promoted and an intracellular signaling system by the radicals is stimulated, which may induce oxidative stress on biomolecules such as DNA, proteins, and lipids, and as a result, damage to skin tissue may occur.

When the oxidative stress on skin cells increases, stimulation occurs in keratinocytes of the epidermis or fibroblasts of the dermis, expression of genes such as matrix metalloproteinase (MMP), which is a collagen-degrading enzyme, may increase through a series of intracellular signal transduction processes, and a decrease in collagen that accounts for 90% of the dermis as a main component of the skin and protects the skin from an external stimulus or force by providing strength and tension to the skin is induced. Accordingly, skin aging or wrinkles may occur. Therefore, effects of preventing aging of skin cells and reducing wrinkles may be expected by regulation of the expression of genes involved in synthesis or degradation of fiber proteins such as collagen.

The aging process of the cells caused by the irradiation of ultraviolet rays in sunlight not only causes cosmetic problems due to damage to skin, but also causes serious health problems such as cancer due to DNA damage of the cells, damage to the central and peripheral nervous system, destruction of the immune system, reproductive organ disorders, developmental disorders of infants and toddlers, and skin diseases, for example, chloracne, and thus, a solution for these problems is urgent.

In order to solve these problems, various materials have been developed, and attempts have been made to overcome these problems by using extracts obtained from various plants or microorganisms. However, in many cases, an exact composition of the extract is unknown since it is not possible to specifically identify which substances in extracts obtained from natural products may have an effect on protection of skin cells or recovery from aging, and thus, extract compositions containing unknown substances are also used together. Due to characteristics of the substance that should be used in direct contact with or applied to a human body, side effects of the material should be minimized and safety for the human body should be ensured. Therefore, a single substance having an effect that may solve the above problems needs to be accurately identified and applied to human skin cells.

### [PRIOR ART DOCUMENTS]

### [Non Patent Document]

(Non Patent Document 1) S Edgar et al., Effects of collagen-derived bioactive peptides and natural antioxidant compounds on proliferation and matrix protein synthesis by cultured normal human dermal fibroblasts, Scientific Reports, 2018; 8:10474

### DISCLOSURE OF THE INVENTION

### TECHNICAL PROBLEM

An objective of the present invention is to provide a peptide having anti-aging activity.

Another objective of the present invention is to provide a composition for inhibiting skin aging or regenerating skin containing the peptide having anti-aging activity as an active ingredient.

Still another objective of the present invention is to provide a cosmetic composition for inhibiting skin aging or regenerating skin containing the peptide having anti-aging activity as an active ingredient.

Still another objective of the present invention is to provide a pharmaceutical composition for preventing or treating photoaging containing the peptide having anti-aging activity as an active ingredient.

### TECHNICAL SOLUTION

In order to achieve the above objectives, an aspect of the present invention provides a peptide containing an amino acid sequence of SEQ ID NO: 1.

Another aspect of the present invention provides a composition for inhibiting skin aging or regenerating skin containing the peptide having anti-aging activity as an active ingredient.

Still another aspect of the present invention provides a cosmetic composition for inhibiting skin aging or regenerating skin containing the peptide having anti-aging activity as an active ingredient.

Still another aspect of the present invention provides a pharmaceutical composition for preventing or treating photoaging containing the peptide having anti-aging activity as an active ingredient.

### ADVANTAGEOUS EFFECTS

The peptide containing an amino acid sequence of SEQ ID NO: 1 according to the present invention has anti-aging activity, and may thus be used as a raw material of a cosmetic for inhibiting skin aging or regenerating skin or a pharmaceutical product for preventing, ameliorating, or treating photoaging.

The effects of the present invention are not limited to the effects mentioned above, and other effects that are not mentioned may be obviously understood by those skilled in the art from the following description.

### BRIEF DESCRIPTION OF THE DRAWINGS

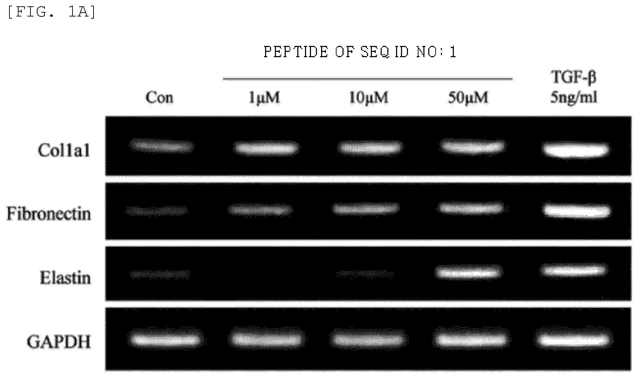

FIG. 1A is a view illustrating a result of confirming expression of Collal, fibronectin, and elastin when fibroblasts are treated with a peptide of the present invention, in which Con represents an untreated group, and TGF-β1 represents a positive control.

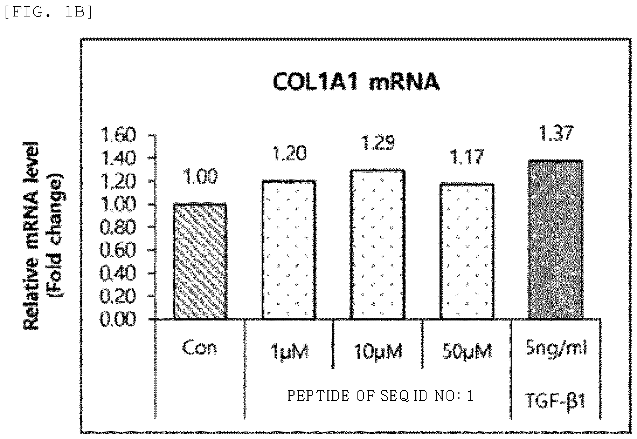

FIG. 1B is a graph showing an effect on expression of Col1a1 when fibroblasts are treated with the peptide of the present invention, in which Con represents an untreated group, and TGF-β1 represents a positive control.

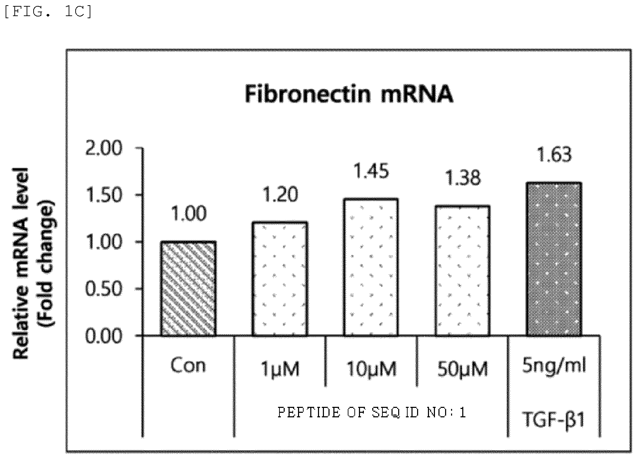

FIG. 1C is a graph showing an effect on expression of fibronectin when fibroblasts are treated with the peptide of the present invention, in which Con represents an untreated group, and TGF-β1 represents a positive control.

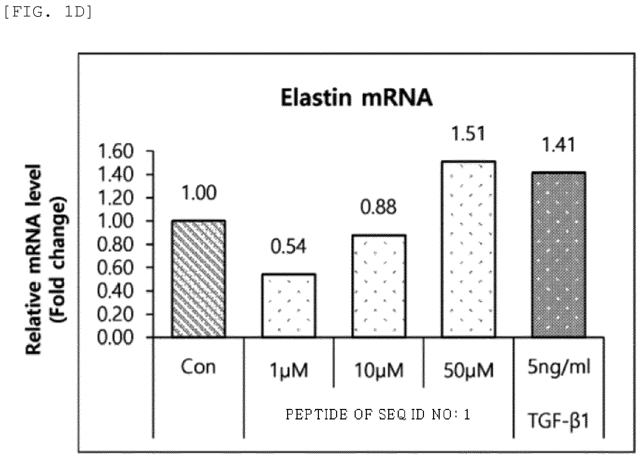

FIG. 1D is a graph showing an effect on expression of elastin when fibroblasts are treated with the peptide of the present invention, in which Con represents an untreated group, and TGF-β1 represents a positive control.

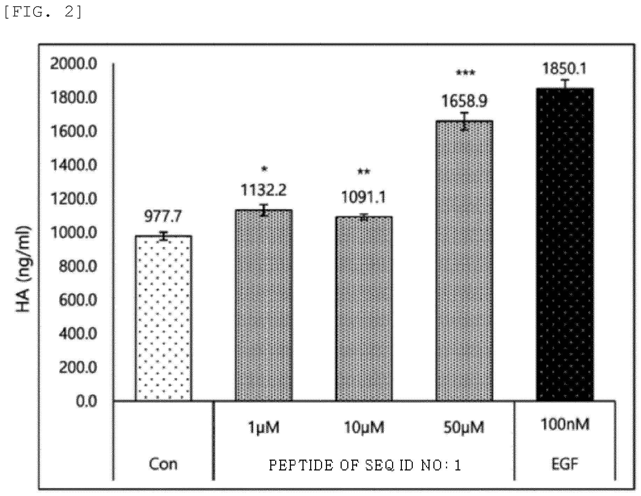

FIG. 2 is a graph showing an effect on secretion of hyaluronic acid protein when keratinocytes are treated with the peptide of the present invention, in which Con represents an untreated group, and EGF represents a positive control.

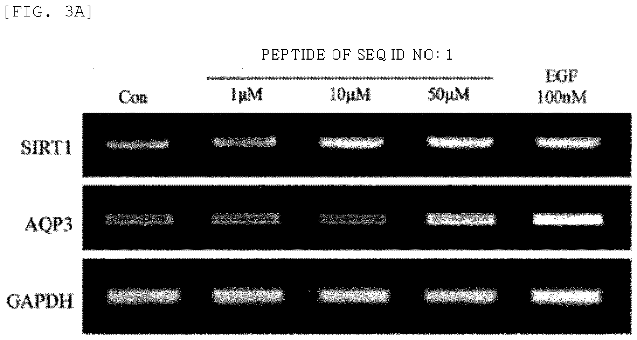

FIG. 3A is a view illustrating a result of confirming expression of AQP3 and SIRT1 when keratinocytes are treated with the peptide of the present invention, in which Con represents an untreated group, and EGF represents a positive control.

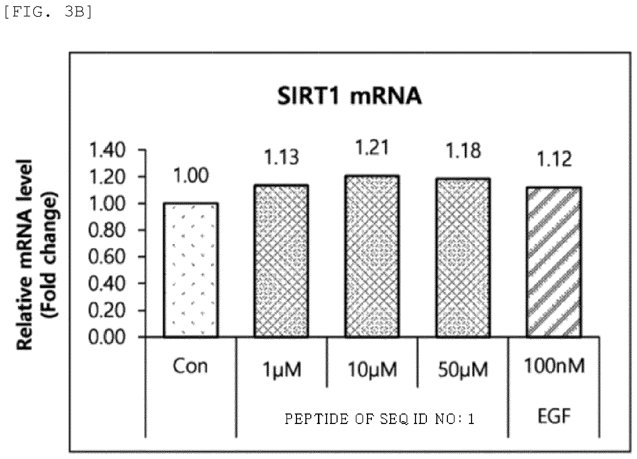

FIG. 3B is a graph showing an effect on expression of SIRT1 when keratinocytes are treated with the peptide of the present invention, in which Con represents an untreated group, and EGF represents a positive control.

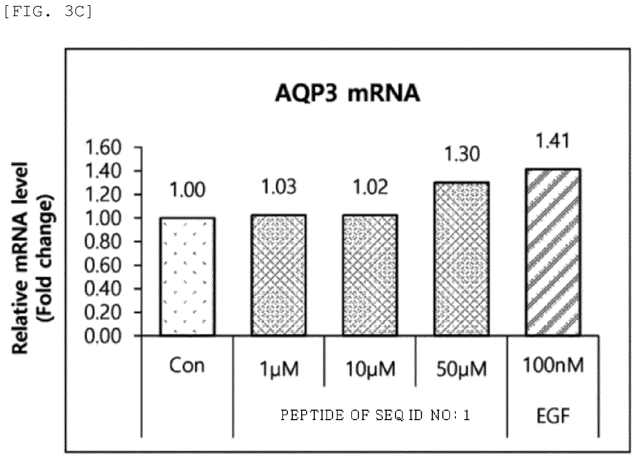

FIG. 3C is a graph showing an effect on expression of AQP3 when keratinocytes are treated with the peptide of the present invention, in which Con represents an untreated group, and EGF represents a positive control.

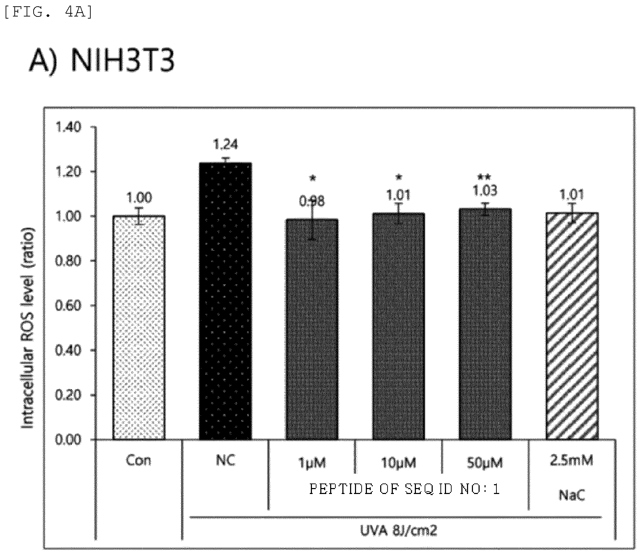

FIG. 4A is a graph showing an effect of the peptide of the present invention on intracellular reactive oxygen species (ROS) levels increased by ultraviolet (UV) irradiation in fibroblasts, in which Con represents a non-UV irradiated group, NC represents a UV irradiated group, 1 µM, 10 µM, and 50 µM represent groups irradiated with ultraviolet rays and treated with the peptide of the present invention at different concentrations, and NaC represents a positive control irradiated with ultraviolet rays and treated with N-Acetylcysteine.

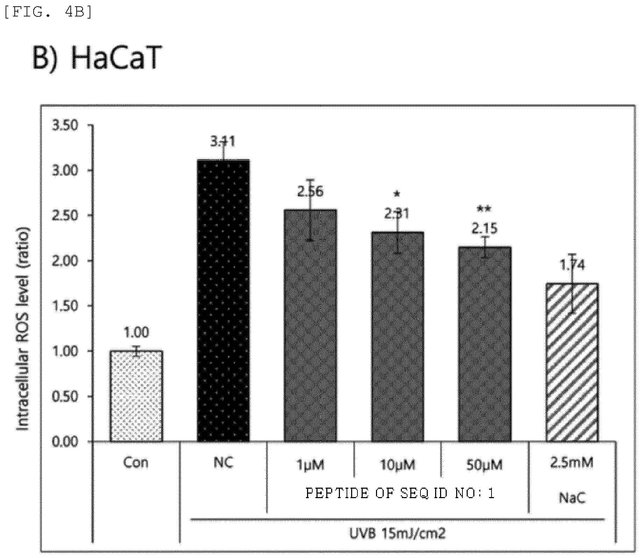

FIG. 4B is a graph showing an effect of the peptide of the present invention on intracellular reactive oxygen species (ROS) levels increased by ultraviolet (UV) irradiation in keratinocytes, in which Con represents a non-UV irradiated group, NC represents a UV irradiated group, 1 µM, 10 µM, and 50 µM represent groups irradiated with ultraviolet rays and treated with the peptide of the present invention at different concentrations, and NaC represents a positive control irradiated with ultraviolet rays and treated with N-Acetylcysteine.

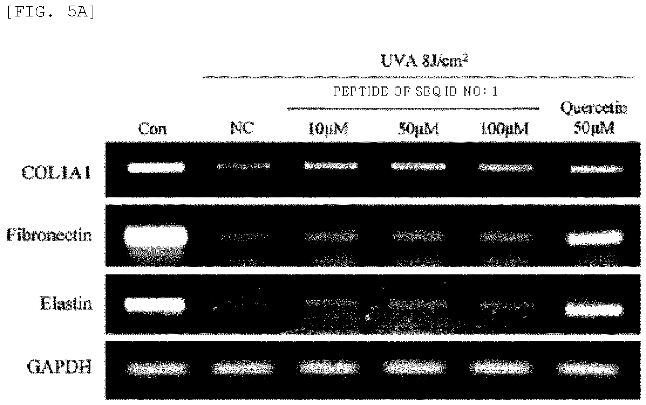

FIG. 5A is a view illustrating a result of confirming an effect of the peptide of the present invention on expression of Collal, fibronectin, and elastin genes decreased by ultraviolet (UV) irradiation in fibroblasts, in which Con represents a non-UV irradiated group, NC represents a UV irradiated group, 1 µM, 10 µM, and 50 µM represent groups irradiated with ultraviolet rays and treated with the peptide of the present invention at different concentrations, and Quercetin represents a positive control irradiated with ultraviolet rays and treated with Quercetin.

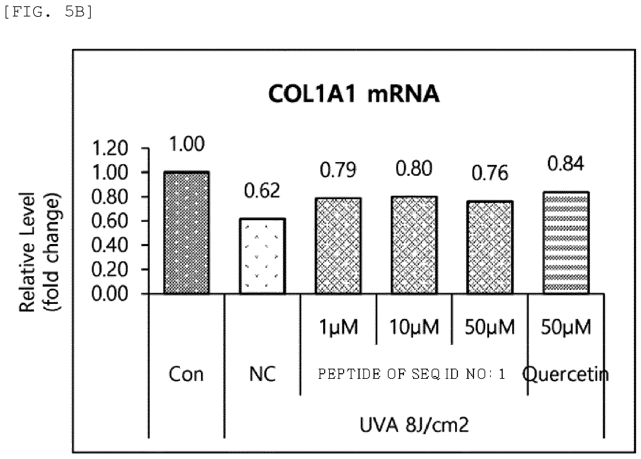

FIG. 5B is a graph showing an effect of the peptide of the present invention on expression of Col1a1 gene decreased by ultraviolet (UV) irradiation in fibroblasts, in which Con represents a non-UV irradiated group, NC represents a UV irradiated group, 1 µM, 10 µM, and 50 µM represent groups irradiated with ultraviolet rays and treated with the peptide of the present invention at different concentrations, and Quercetin represents a positive control irradiated with ultraviolet rays and treated with Quercetin.

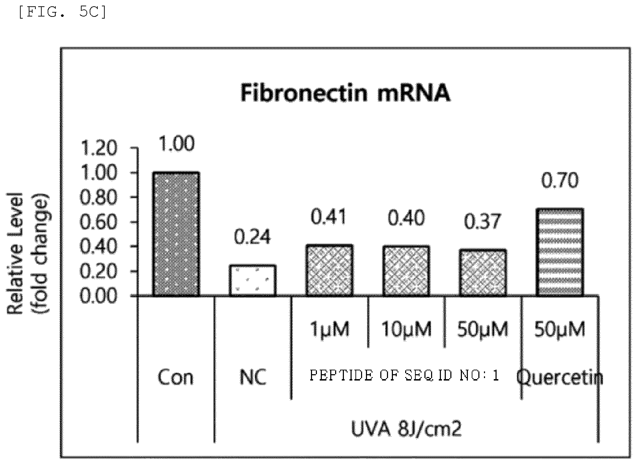

FIG. 5C is a graph showing an effect of the peptide of the present invention on expression of fibronectin gene decreased by ultraviolet (UV) irradiation in fibroblasts, in which Con represents a non-UV irradiated group, NC represents a UV irradiated group, 1 µM, 10 µM, and 50 µM represent groups irradiated with ultraviolet rays and treated with the peptide of the present invention at different concentrations, and Quercetin represents a positive control irradiated with ultraviolet rays and treated with Quercetin.

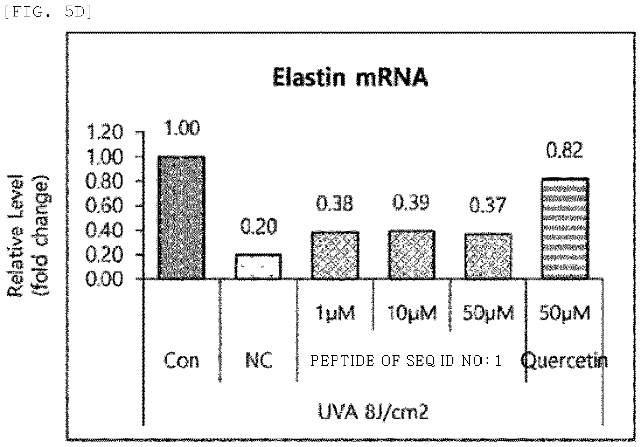

FIG. 5D is a graph showing an effect of the peptide of the present invention on expression of elastin gene decreased by ultraviolet (UV) irradiation in fibroblasts, in which Con represents a non-UV irradiated group, NC represents a UV irradiated group, 1 µM, 10 µM, and 50 µM represent groups irradiated with ultraviolet rays and treated with the peptide of the present invention at different concentrations, and Quercetin represents a positive control irradiated with ultraviolet rays and treated with Quercetin.

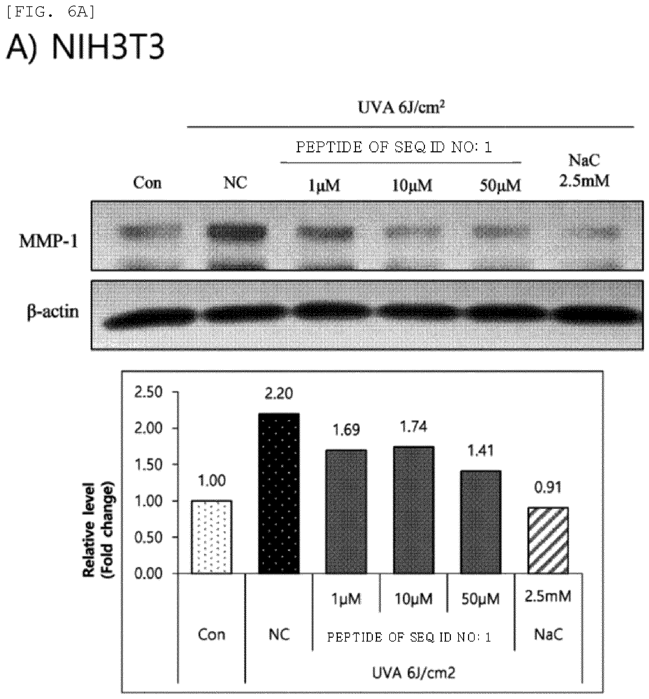

FIG. 6A is a graph showing an effect of the peptide of the present invention on expression of MMP-1 increased by ultraviolet (UV) irradiation in fibroblasts, in which Con represents a non-UV irradiated group, NC represents a UV irradiated group, 1 µM, 10 µM, and 50 µM represent groups irradiated with ultraviolet rays and treated with the peptide of the present invention at different concentrations, and NaC represents a positive control irradiated with ultraviolet rays and treated with N-Acetylcysteine.

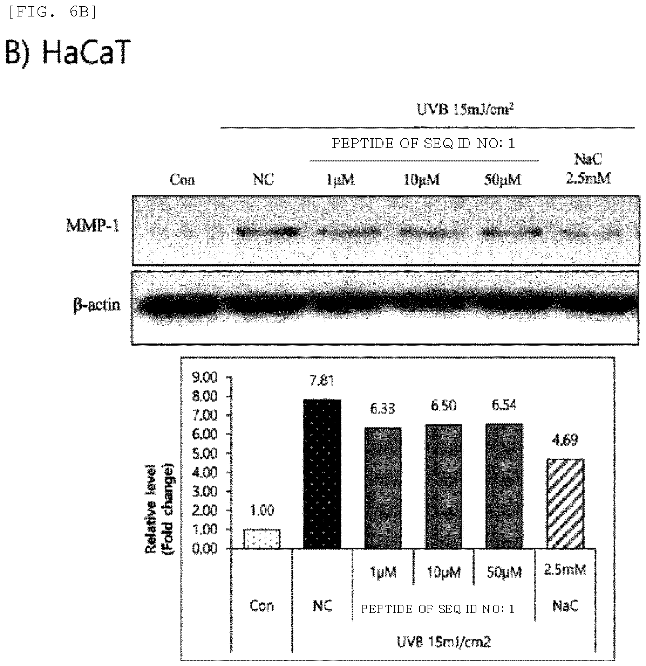

FIG. 6B is a graph showing an effect of the peptide of the present invention on expression of MMP-1 increased by ultraviolet (UV) irradiation in keratinocytes, in which Con represents a non-UV irradiated group, NC represents a UV irradiated group, 1 µM, 10 µM, and 50 µM represent groups irradiated with ultraviolet rays and treated with the peptide of the present invention at different concentrations, and NaC represents a positive control irradiated with ultraviolet rays and treated with N-Acetylcysteine.

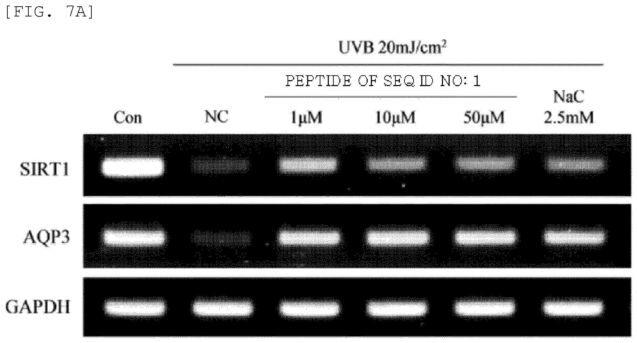

FIG. 7A is a view illustrating a result of confirming an effect of the peptide of the present invention on mRNA levels of AQP3 and SIRT1 decreased by ultraviolet (UV) irradiation in keratinocytes, in which Con represents a non-UV irradiated group, NC represents a UV irradiated group, 1 µM, 10 µM, and 50 µM represent groups irradiated with ultraviolet rays and treated with the peptide of the present invention at different concentrations, and NaC represents a positive control irradiated with ultraviolet rays and treated with N-Acetylcysteine.

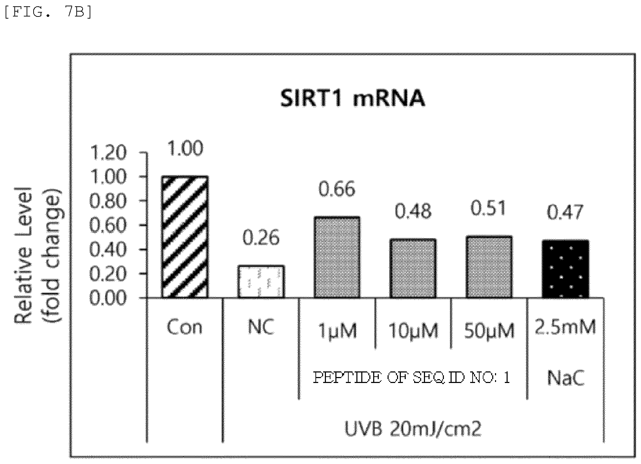

FIG. 7B is a graph showing an effect of the peptide of the present invention on an mRNA level of SIRT1 decreased by ultraviolet (UV) irradiation in keratinocytes, in which Con represents a non-UV irradiated group, NC represents a UV irradiated group, 1 µM, 10 µM, and 50 µM represent groups irradiated with ultraviolet rays and treated with the peptide of the present invention at different concentrations, and NaC represents a positive control irradiated with ultraviolet rays and treated with N-Acetylcysteine.

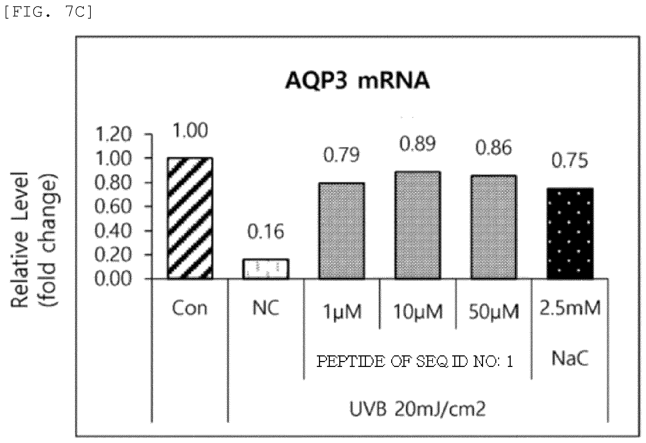

FIG. 7C is a graph showing an effect of the peptide of the present invention on an mRNA level of AQP3 decreased by ultraviolet (UV) irradiation in keratinocytes, in which Con represents a non-UV irradiated group, NC represents a UV irradiated group, 1 µM, 10 µM, and 50 µM represent groups irradiated with ultraviolet rays and treated with the peptide of the present invention at different concentrations, and NaC represents a positive control irradiated with ultraviolet rays and treated with N-Acetylcysteine.

### MODE FOR CARRYING OUT THE INVENTION

Hereinafter, the present invention will be described in more detail.

An aspect of the present invention provides a peptide containing an amino acid sequence set forth in SEQ ID NO: 1.

In the present specification, the term "peptide" refers to a linear molecule consisting of amino acid residues, and specifically, the peptide of the present invention may contain an amino acid sequence set forth in SEQ ID NO: 1.

The "peptide" may be variants or fragments of amino acids having different sequences by deletion, insertion, substitution, or combination of amino acid residues within a range that does not affect the function. Amino acid exchanges that do not entirely alter the activity of the peptide are known in the art. In some cases, the peptide may be modified by phosphorylation, sulfation, acrylation, glycosylation, methylation, farnesylation, or the like. Accordingly, the peptide of the present invention includes a peptide containing substantially the same amino acid sequence as the peptide containing an amino acid sequence of SEQ ID NO: 1, a variant thereof, or an active fragment thereof.

The substantially identical protein refers to an amino acid sequence having 75% or more, preferably 80% or more, for example, 85% or more, 90% or more, 95% or more, 98% or more, or 99% or more sequence homology with the amino acid sequence of SEQ ID NO: 1, but is not limited thereto, and it is included in the scope of the present invention as long as it has 75% or more amino acid sequence homology and has the same activity.

In addition, the peptide of the present invention may further contain a targeting sequence, a tag, a labeled residue, or an amino acid sequence prepared for a specific purpose to increase half-life or stability of the peptide.

In addition, in order to obtain better chemical stability, enhanced pharmacological properties (half-life, absorptivity, potency, efficacy, and the like), altered specificity (for example, a broad spectrum of biological activity), and reduced antigenicity, the N-terminus or the C-terminus of the peptide of the present invention may be modified. The "stability" is used to include not only stability in vivo for protecting the peptide of the present invention from attack by a protease in vivo, but also storage stability (for example, storage stability at room temperature).

The modification of the N-terminus may be binding of a protecting group selected from the group consisting of an acetyl group, a fluoreonylmethoxycarbonyl group, a formyl group, a palmitoyl group, a myristyl group, a stearyl group, and polyethylene glycol (PEG), to the N-terminus of the peptide, but is not limited thereto.

The modification of the C-terminus may be binding of a hydroxyl group (-OH), an amino group (-NH₂), azide (-NHNH₂), or the like, to the C-terminus of the peptide, but is not limited thereto.

Synthesis of the peptide of the present invention may be performed, for example, using a device or a genetic engineering technique. In a case where the peptide is synthesized using a device, a desired peptide may be synthesized using an Fmoc solid-phase method in an automatic peptide synthesizer.

In a specific embodiment of the present invention, the peptide containing the amino acid sequence of SEQ ID NO: 1 of the present invention is synthesized and identified using the Fmoc solid-phase method, and is selected by verifying efficacy of the identified peptide.

In a specific embodiment of the present invention, in order to verify the efficacy of the identified peptide, the peptide of the present invention is selected through validation of the efficacy of the peptide for an effect of increasing expression levels of an extracellular matrix (ECM) component and a skin barrier strengthening factor, an effect of decreasing the reactive oxygen species level increased by ultraviolet irradiation, and an effect of inhibiting expression of a collagen degradation-related protein increased by ultraviolet rays.

Accordingly, the peptide containing the amino acid sequence of SEQ ID NO: 1 of the present invention has skin aging inhibition activity or skin regeneration activity.

Another aspect of the present invention provides a composition for skin aging inhibition or skin regeneration containing the peptide containing the amino acid sequence of SEQ ID NO: 1 as an active ingredient.

The "skin aging" may be aging that occurs naturally in cells over time, or may be photoaging caused by sunlight. In addition, the aging may be induced by an intracellular oxidative stress, which may occur due to various causes, and accordingly, cell growth inhibition or apoptosis may occur, synthesis of various fiber proteins constituting skin cells may be inhibited, and expression of a degrading enzyme may increase. In particular, when ultraviolet rays are irradiated, expression of gene such as Collal, fibronectin, elastin, or hyaluronic acid is inhibited in skin cells, which may cause photoaging of skin cells and wrinkles.

The "skin regeneration" may promote growth of skin cells and enhance viability of skin cells to prevent cell damage from an external and/or internal stimulus, reduce wrinkles in the skin, improve elasticity, and strengthen the skin barrier, and the skin regeneration includes prevention of skin damage caused by the external environment, for example, pigmentation, or amelioration or treatment of skin photoaging.

The composition for skin aging inhibition or skin regeneration improves resistance to cell damage caused by aging or an external stimulus.

The "external stimulus" may be a physical stimulus, a chemical stimulus caused by applying cosmetics or other external preparations, a stimulus by ultraviolet rays, or a stimulus by infrared rays.

The damaged cells may be skin fibroblasts or keratinocytes.

The peptide of the present invention may induce an increase in expression of a fiber protein synthesis gene in skin cells. Therefore, the peptide may reduce wrinkles of the skin and improve elasticity of the skin by increasing the synthesis of fiber proteins, and may increase resistance and viability of skin cells and enhance a regenerative ability of skin damaged by an external stimulus or aging by improving the skin barrier. The skin aging inhibition and skin regeneration effects of the peptide of the present invention may be confirmed by measuring expression levels of proteins or anti-aging genes involved in cell proliferation, and fiber proteins, whose expression levels change within skin cells as skin aging progresses.

The composition for skin aging inhibition or skin regeneration may increase expression or secretion of collagen, fibronectin, elastin, or hyaluronic acid in skin cells.

The composition for skin aging inhibition or skin regeneration may increase expression of AQP3 or SIRT1, which is a skin barrier strengthening factor.

The composition for skin aging inhibition or skin regeneration may inhibit production of reactive oxygen species (ROS) caused by ultraviolet rays.

The composition for skin aging inhibition or skin regeneration may increase expression of collagen, fibronectin, or elastin decreased by ultraviolet rays.

The composition for skin aging inhibition or skin regeneration may inhibit expression of MMP-1 increased by ultraviolet rays.

In a specific embodiment of the present invention, the peptide containing the amino acid sequence of SEQ ID NO: 1 of the present invention is shown to increase expression of genes encoding extracellular matrix proteins such as Collal, fibronectin, and elastin in fibroblasts.

In addition, the peptide is shown to increase secretion of hyaluronic acid in keratinocytes.

In addition, the peptide is shown to increase expression of skin barrier strengthening genes such as AQP3 and SIRT1 in keratinocytes.

In addition, the peptide is shown to decrease an intracellular reactive oxygen species (ROS) level in fibroblasts and/or keratinocytes damaged by ultraviolet rays.

In addition, the peptide is shown to increase expression of Collal, fibronectin, and elastin genes decreased in fibroblasts damaged by ultraviolet rays.

In addition, the peptide is shown to inhibit expression of MMP-1 increased in fibroblasts and/or keratinocytes damaged by ultraviolet rays.

In addition, the peptide is shown to increase mRNA levels of SIRT1 and AQP3 decreased by ultraviolet rays.

Therefore, it is clear that the peptide of the present invention has skin aging inhibition or skin regeneration activity by regulating the expression of genes related to the aging phenomenon of skin cells, and the peptide of the present invention may be efficiently used as an active ingredient of a composition for skin aging inhibition or skin regeneration.

Still another aspect of the present invention provides a cosmetic composition for skin aging inhibition or skin regeneration containing the peptide containing the amino acid sequence of SEQ ID NO: 1 as an active ingredient.

The skin aging inhibition or skin regeneration may be prevention or reduction of skin wrinkles, skin elasticity improvement, skin barrier strengthening, prevention of pigmentation, or amelioration of skin photoaging.

The peptide containing the amino acid sequence of SEQ ID NO: 1 is the same as the peptide described in the "composition for skin aging inhibition or skin regeneration", and thus the detailed description thereof refers to the content described above. Hereinafter, only a specific configuration of the cosmetic composition for skin aging inhibition or skin regeneration will be described.

The cosmetic composition may be prepared into any formulation commonly prepared in the art, for example, a solution, a suspension, an emulsion, a paste, a gel, a cream, a lotion, a powder, a soap, a surfactant-containing cleanser, an oil, a powder foundation, an emulsion foundation, a wax foundation, and a spray, but is not limited thereto.

The cosmetic composition may be prepared in various forms such as a solution, a sol gel, an emulsion, an oil, a wax, and an aerosol, for example, a soft lotion, a nourishing lotion, a nourishing cream, a massage cream, an essence, an eye cream, a cleansing cream, a cleansing foam, a cleansing water, a pack, a spray, a powder, a hair tonic, a hair cream, a hair lotion, a hair shampoo, a hair conditioner, a hair conditioner, a hair spray, a hair aerosol, a pomade, a gel, and the like, but is not limited thereto.

The cosmetic composition of the present invention may contain other additives such as an excipient and a carrier, and it is possible to apply and blend usual ingredients that are combined to general skin cosmetics as needed.

In a case where the formulation of the cosmetic composition of the present invention is a paste, cream, or gel, animal oil, vegetable oil, wax, paraffin, starch, tragacanth, a cellulose derivative, polyethylene glycol, silicone, bentonite, silica, talc, or zinc oxide may be used as a carrier ingredient.

In a case where the formulation of the cosmetic composition is a powder or spray, lactose, talc, silica, aluminum hydroxide, calcium silicate, or polyamide powder may be used as a carrier ingredient, and in particular, in a case where the formulation of the cosmetic composition is a spray, a propellant such as chlorofluorohydrocarbon, propane/butane, or dimethyl ether may be additionally contained, but is not limited thereto.

In a case where the formulation of the cosmetic composition is a solution or emulsion, a solvent, a solubilizer, or an emulsifier may be used as a carrier ingredient, for example, water, ethanol, isopropanol, ethyl carbonate, ethyl acetate, benzyl alcohol, benzyl benzoate, propylene glycol, 1,3-butylglycol oil, glycerol aliphatic ester, polyethylene glycol, fatty acid ester of sorbitan, or the like may be used.

In a case where the formulation of the cosmetic composition is a suspension, a liquid diluent such as water, ethanol, or propylene glycol, a suspending agent such as ethoxylated isostearyl alcohol, polyoxyethyl sorbitol ester, or polyoxyethylene sorbitan ester, microcrystalline cellulose, aluminum metahydroxide, bentonite, agar, tragacanth, or the like may be used.

In a case where the formulation of the cosmetic composition is a surfactant-containing cleanser, aliphatic alcohol sulfate, aliphatic alcohol ether sulfate, sulfosuccinic acid monoester, isethionate, an imidazolinium derivative, methyltaurate, sarcosinate, fatty acid amide ether sulfate, alkylamidobetaine, aliphatic alcohol, fatty acid glyceride, fatty acid diethanolamide, vegetable oil, a lanolin derivative, ethoxylated glycerol fatty acid ester, or the like may be used as a carrier ingredient.

In a case where the formulation of the cosmetic composition is a hair shampoo, a base ingredient for forming the shampoo, such as a thickener, a surfactant, a viscosity modifier, a moisturizer, a pH adjuster, a preservative, or an essential oil, may be mixed with the peptide of the present invention. CDE may be used as the thickener, LES, which is an anionic surfactant, or coco betain, which is an amphoteric surfactant, may be used as the surfactant, polyquaternium may be used as the viscosity modifier, glycerin may be used as the moisturizer, and citric acid or sodium hydroxide may be used as the pH adjuster. A grapefruit extract or the like may be used as the preservative, and in addition, an essential oil such as cedarwood, peppermint, or rosemary, silk amino acid, pentanol, or vitamin E may be added.

The ingredients contained in the cosmetic composition may further include, in addition to the peptide of the present invention and the carrier ingredient, ingredients commonly used in a cosmetic composition, for example, common adjuvants such as an antioxidant, a stabilizer, a solubilizer, a vitamin, a pigment, and a perfume, as active ingredients, but are not limited thereto.

Still another aspect of the present invention provides a pharmaceutical composition for preventing or treating photoaging containing the peptide containing the amino acid sequence of SEQ ID NO: 1 as an active ingredient.

The peptide containing the amino acid sequence of SEQ ID NO: 1 is the same as the peptide described in the "composition for skin aging inhibition or skin regeneration", and thus the detailed description thereof refers to the content described above. Hereinafter, only a specific configuration of the pharmaceutical composition for preventing or treating photoaging will be described.

In the present specification, the "photoaging" refers to a phenomenon of skin damage, pigmentation, wrinkles, or deterioration of skin elasticity that occurs due to repeated or long-term exposure to sunlight.

In the present specification, the "prevention" refers to any action that inhibits or delays the occurrence, spread, and recurrence of photoaging by the peptide of the present invention or a composition containing the same.

In the present specification, the "amelioration or treatment" refers to any action that beneficially changes photoaging, such as delaying, stopping, or reversing the progression of photoaging by the peptide of the present invention or a composition containing the same.

In a specific embodiment of the present invention, the peptide increases expression or secretion of extracellular matrix (ECM) proteins such as collagen, fibronectin, elastin, and/or hyaluronic acid, and increases expression of skin barrier strengthening factors such as AQP3 and/or SIRT1, such that photoaging may be prevented, ameliorated, or treated.

In addition, the peptide inhibits production of reactive oxygen species (ROS) in cells damaged by aging or an external stimulus, increases decreased expression of collagen, fibronectin, and/or elastin, inhibits increased expression of MMP-1, and increases decreased expression of skin barrier strengthening factors such as AQP3 and/or SIRT1, such that photoaging may be prevented, ameliorated, or treated.

The pharmaceutical composition of the present invention may further contain a pharmaceutically acceptable carrier.

The pharmaceutical composition may further contain, for example, a carrier for oral administration or a carrier for parenteral administration, as the pharmaceutically acceptable carrier. The carrier for oral administration may include lactose, starch, a cellulose derivative, magnesium stearate, stearic acid, and the like. In addition, the carrier for parenteral administration may include water, suitable oil, a saline solution, aqueous glucose, glycol, and the like. In addition, the pharmaceutical composition may further contain a stabilizer and a preservative. Suitable stabilizers include antioxidants such as sodium bisulfite, sodium sulfite, and ascorbic acid. Suitable preservatives include benzalkonium chloride, methyl- or propyl-paraben, and chlorobutanol. For suitable pharmaceutically acceptable carriers, those described in Remington's Pharmaceutical Sciences (19th ed., Mack Publishing Company, Easton, PA, 1995) may be referred to.

The pharmaceutical composition of the present invention may be administered to mammals including a human by any method. For example, the pharmaceutical composition of the present invention may be administered orally or parenterally. The parental administration may be, but is not limited to, intravenous, intramuscular, intraarterial, intramedullary, intradural, intracardiac, transdermal, subcutaneous, intraperitoneal, intranasal, intestinal, topical, sublingual, or rectal administration.

The pharmaceutical composition of the present invention may be formulated into a preparation for oral administration or a preparation for parenteral administration according to a route of administration as described above, and the preparation for parenteral administration may be specifically formulated and used in the form of a preparation for injection or an external preparation.

The pharmaceutical composition of the present invention is administered in a pharmaceutically effective amount. In the present invention, the "pharmaceutically effective amount" refers to an amount sufficient to treat a disease with a reasonable benefit/risk ratio applicable for medical treatment. An effective dose level may be determined according to factors including the type of disease and severity of a patient, activity of a drug, sensitivity to a drug, an administration time, a route of administration, an emission rate, a duration of treatment, and drugs used simultaneously, and other factors well-known in the medical field. The pharmaceutical composition may be administered as a separate therapeutic agent or in combination with another therapeutic agent, may be administered simultaneously, separately, or sequentially with a conventional therapeutic agent, or may be administered singly or multiply. Considering all the factors, it is important to administer the pharmaceutical composition in an amount capable of obtaining a maximum effect with a minimal amount without side-effects, and the amount of the pharmaceutical composition to be administered may be easily determined by those skilled in the art.

The effective amount of the pharmaceutical composition may be changed according to the patient's age, gender, condition, weight, absorbency of the active ingredient in vivo, inactivation rate, excretion rate, disease type, and drug used in combination, and may be increased or decreased according to the route of administration, the severity, the gender, the weight, the age, or the like. For example, the pharmaceutical composition may be administered at about 0.0001 µg to 500 mg, preferably, 0.01 µg to 100 mg per 1 kg of the patient's weight per day.

As described above, the peptide of the present invention has excellent skin aging inhibition and skin regeneration effects, and may thus be used as a raw material of a cosmetic for inhibiting skin aging or regenerating skin or a pharmaceutical product for preventing, ameliorating, or treating photoaging.

Still another aspect of the present invention provides a method for inhibiting skin aging or regenerating skin, the method including administering, to a subject, a therapeutically effective amount of a peptide containing an amino acid sequence of SEQ ID NO: 1.

Still another aspect of the present invention provides a method for preventing or treating photoaging, the method including administering, to a subject, a therapeutically effective amount of a peptide containing an amino acid sequence of SEQ ID NO: 1.

The peptide containing the amino acid sequence of SEQ ID NO: 1 is the same as the peptide described in the "composition for skin aging inhibition or skin regeneration", and thus the detailed description thereof refers to the content described above.

In the present specification, the term "therapeutically effective amount" refers to the amount of peptide that achieves the goal of reducing the frequency of disease onset during treatment with the peptide, but avoids harmful side effects usually associated with other treatments.

Still another aspect of the present invention provides a use of the amino acid sequence of SEQ ID NO: 1 for use in preparation of a drug for preventing or treating photoaging.

Hereinafter, the present invention will be described in detail with reference to Examples.

However, the following Examples illustrate only the present invention, and the present invention is not limited by the following Examples.

### Mode for Invention

### [Synthesis Example 1]

### Synthesis of Peptide Containing Amino Acid Sequence of SEQ ID NO: 1

A peptide containing an amino acid sequence of SEQ ID NO: 1 of [Table 1] was synthesized by using an automatic peptide synthesizer (Liberty, CEM Corporation, USA), and then, the synthesized peptide was isolated and purified using C18 reverse-phase high-performance liquid chromatography (HPLC) (U-3000, Thermo fisher scientific, USA). Pursuit XRs C18 (250 * 4.65 mm 100 Å, Agilent, USA) was used as a column.

**[Table 1]**

| SEQ ID NO: | Amino acid sequence of peptide |
|---|---|
| 1 | LCACCLHNCNECQ |

### [Experimental Example 1] Confirmation of Inhibition of Natural Aging by Peptide Treatment

### 1-1. Confirmation of Increase in Expression of Extracellular Matrix (ECM) Components (Collagen, Fibronectin, and Elastin) Genes

Expression levels of genes involved in natural aging in fibroblasts were measured to confirm whether the peptide of the present invention inhibited the cell aging phenomenon that occurs naturally in cells.

First, NIH3T3 cells (mouse fibroblast cell line) were seeded in a 6-well plate at a cell density of 3 × 10⁵ cell/well, and then the cells were cultured in DMEM medium containing 10% fetal bovine serum (FBS) for 24 hours. The cultured cells were washed once with serum free DMEM medium, and the peptide of the present invention was mixed in 1 mL of the serum free DMEM medium at each of concentrations of 1, 10, and 50 µM, and dispensed into the cells. A positive control (PC) was treated with 5 ng/ml of TGF-β1 instead of the above peptide. The cells were cultured in a CO₂ incubator at 37°C for 24 hours.

The cultured cells were washed twice with PBS, and RNA was extracted from the cells using easy blue (iNtRON, Cat. No.: 17061, Korea). The amount of RNA extracted was quantified, 1,000 ng of RNA was added per tube, and RT reaction was performed using a kit (enzynomics, Cat. No.: PT200, Korea). RT-PCR was performed using a PCR kit (enzynomics, Cat. No.: P581T, Korea). The sequences of the primers of collagen, fibronectin, and elastin used in RT-PCR and the sequence of the primer of GAPDH used to compare the total RNA amount of each treatment group are shown in [Table 2].

**[Table 2]**

| **Gene** | **Primer** | **Sequence (5' ->3')** | **SEQ ID NO:** |
|---|---|---|---|
| Col1a1 | F | CAC CCT CAA GAG CCT GAG TC | 2 |
| | R | AGA CGG CTG AGT AGG GAA CA | 3 |
| Fibronectin | F | CCA GGA ACC GAG TAC ACC AT | 4 |
| | R | ATA CCC AGG TTG GGT GAT GA | 5 |
| Elastin | F | GCAAGACCTGGCTTTGGACT | 6 |
| | R | GGGAGTTTCTGGTTAGGGCTG | 7 |
| SIRT1 | F | TCA GTG GGT GGA ACA GTG AG | 8 |
| | R | TCT GGC ATG TCC CAC TAT CA | 9 |
| AQP3 | F | CCT TCT TGG GTG CTG GAA TA | 10 |
| | R | ACA GGA TAA GGG AGG CTG TG | 11 |
| GAPDH | F | GGA GCC AAA AGG GTG ATC AT | 12 |
| | R | GTG ATG GCA TGG ACT GTG GT | 13 |

As a result, as illustrated in FIGS. 1A to 1D, it was confirmed that the expression of the collagen, fibronectin, and elastin genes gradually increased as a concentration of the peptide increased when treated with the peptide. From this, it may be appreciated that as the expression of collagen, fibronectin, and elastin, which are extracellular matrix proteins that constitute dermis of fibroblasts, increases by the peptide containing the amino acid sequence of SEQ ID NO: 1, the effect of inhibiting or ameliorating natural aging such as skin wrinkles is obtained.

### 1-2. Confirmation of Increase in Expression of Extracellular Matrix (ECM) Component (Hyaluronic Acid) Gene

An expression level of gene involved in natural aging in keratinocytes was measured to confirm whether the peptide of the present invention inhibited the cell aging phenomenon that occurs naturally in cells.

First, HaCaT cells (human keratinocyte cell line) were seeded in a 48-well plate at a cell density of 1 × 10⁵ cell/well, and then the cells were cultured in DMEM medium containing 10% fetal bovine serum (FBS) for 24 hours. The cultured cells were washed once with serum free DMEM medium, and the peptide of the present invention was mixed in 2 mL of the serum free DMEM medium at each of concentrations of 1, 10, and 50 µM, and dispensed into three wells by 500 µL per condition. A positive control (PC) was treated with 100 nM of EGF instead of the above peptide. The cells were cultured in a CO₂ incubator at 37°C for 24 hours.

The medium of the cultured HaCaT cells was centrifuged to settle a suspended matter, and a supernatant was transferred to an e-tube and used to perform HA ELISA (ECHELON biosciences, Cat No.: K-1200, USA).

As a result, as illustrated in FIG. 2, it was confirmed that secretion of hyaluronic acid protein gradually increased as the concentration of the peptide increased when treated with the peptide.

From this, it may be appreciated that as the amount of hyaluronic acid protein, which is an extracellular matrix protein that constitutes dermis of keratinocytes, is increased by the peptide containing the amino acid sequence of SEQ ID NO: 1, the effect of inhibiting or ameliorating natural aging such as skin wrinkles is obtained.

### 1-3. Confirmation of Increase in Expression of Genes (SIRT1 and AQP3) Related to Skin Barrier Function

Expression levels of genes involved in natural aging in keratinocytes were measured to confirm whether the peptide of the present invention inhibited the cell aging phenomenon that occurs naturally in cells.

First, HaCaT cells were seeded in a 6-well plate at a cell density of 3 × 10⁵ cell/well, and then the cells were cultured in DMEM medium containing 10% fetal bovine serum (FBS) for 24 hours. The cultured cells were washed once with serum free DMEM medium, and then the peptide of the present invention was mixed in 1 mL of the serum free DMEM medium at each of concentrations of 1, 10, and 50 µM, and dispensed into the cells. A positive control (PC) was treated with 100 nM of EGF instead of the above peptide. The cells were cultured in a CO₂ incubator at 37°C for 24 hours.

The cultured cells were washed twice with PBS, and then RNA was extracted from the cells using easy blue (iNtRON, Cat. No.: 17061, Korea). The extracted RNA was subjected to RT reaction using a kit (enzynomics, Cat. No.: RT200, Korea). Thereafter, RT-PCR was performed using a PCR kit (enzynomics, Cat. No.: R581T, Korea). The sequences of the primers of AQP3 and SIRT1 used in RT-PCR and the sequence of the primer of GAPDH used to compare the total RNA amount of each treatment group are shown in [Table 2].

As a result, as illustrated in FIGS. 3A to 3C, it was confirmed that the mRNA levels of AQP3 and SIRT1, which are skin barrier strengthening factors, increased when treated with the peptide.

From this, it may be appreciated that as the expression levels of AQP3 and SIRT1, which are skin barrier strengthening factors, increase in keratinocytes by the peptide containing the amino acid sequence of SEQ ID NO: 1, the effect of inhibiting or ameliorating natural aging is obtained.

### [Experimental Example 2]

### Confirmation of Inhibition of Extrinsic Aging by Peptide Treatment

### 2-1. Confirmation of Effect of Inhibiting Active Oxygen Species Increased by Ultraviolet Rays

The reactive oxygen species level increases when UV is irradiated. Therefore, it was confirmed whether the reactive oxygen species level decreased again and the aging phenomenon caused by oxidative stress in cells was inhibited when treated with the peptide of the present invention.

First, NIH3T3 cells and HaCaT cells were seeded in a 6-well plate at a cell density of 5 × 10⁵ cell/well, respectively, and then the cells were cultured in DMEM medium containing 10% fetal bovine serum (FBS) for 24 hours. The cultured cells were washed once with serum free DMEM medium, and then the peptide of the present invention was mixed in 1 mL of the serum free DMEM medium at each of concentrations of 1, 10, and 50 µM, and dispensed into the cells. A positive control (PC) was treated with 2.5 mM of N-Acetylcysteine (NaC) instead of the above peptide.

The cells were cultured in a CO₂ incubator at 37°C for 1 hour. The medium of the cultured cells was transferred to a tube, 1 ml of PBS was added, and then the NIH3T3 cells were irradiated with 8 J/cm² of UVA and the HaCaT cells were irradiated with 15 mJ/cm² of UVB using a UV irradiator (VILBER LOURMAT, Cat No.: 3102-BSU, France). After removing PBS, 900 µL of the medium transferred to the tube was dispensed, and culture was performed in a CO₂ incubator at 37°C for 24 hours. After treatment with 10 µM of dichloro-dihydro-fluorescein diacetate (DCFH-DA), wrapping with a foil was performed, and culture was performed in a CO₂ incubator at 37°C for 30 minutes. The cells were washed twice with PBS, and 500 µL of 1XTE was dispensed to isolate the cells. After centrifugation, the cells were washed with PBS, and a fluorescence value was measured through FACS.

As a result, as illustrated in FIGS. 4A and 4B, it was confirmed that when the NIH3T3 cells and the HaCaT cells were treated with the peptide of the present invention, the intracellular reactive oxygen species level increased by UV irradiation decreased.

From this, it may be appreciated that the reactive oxygen species level increased by UV irradiation in keratinocytes decreases again by the peptide containing the amino acid sequence of SEQ ID NO: 1, and the oxidative stress is inhibited, and as a result, the photoaging phenomenon of the cells is prevented.

### 2-2. Confirmation of Increase in Expression of Extracellular Matrix Components (Collagen, Fibronectin, and Elastin) Genes Inhibited by Ultraviolet Rays

The expression of genes related to extracellular matrix components is inhibited when irradiated with UV. Therefore, it was confirmed whether the photoaging phenomenon of the cells was inhibited by confirming whether the expression levels of the Collal, fibronectin, and elastin genes were restored and increased again when treated with the peptide of the present invention.

First, NIH3T3 cells were seeded in a 6-well plate at a cell density of 5 × 10⁵ cell/well, and then the cells were cultured in DMEM medium containing 10% fetal bovine serum (FBS) for 24 hours. The cultured cells were washed once with serum free DMEM medium, and then the peptide of the present invention was mixed in 1 mL of the serum free DMEM medium at each of concentrations of 1, 10, and 50 µM, and dispensed into the cells. A positive control (PC) was treated with 50 µM of Quercetin instead of the above peptide. The cells were cultured in a CO₂ incubator at 37°C for 1 hour.

The medium of the cultured cells was transferred to a tube (e-tube), 1 ml of PBS was added, and irradiation was performed with 8 J/cm² of UVA using a UV irradiator (VILBER LOURMAT, Cat No.: 3102-BSU, France). After removing PBS, 900 µL of the medium transferred to the tube was dispensed, and culture was performed in a CO₂ incubator at 37°C for 6 hours. The cultured cells were washed twice with PBS, and then RNA was extracted from the cells using easy blue (iNtRON, Cat. No.: 17061, Korea). The amount of RNA extracted was quantified, 1,000 ng of RNA was added per tube, and RT reaction was performed using a kit (enzynomics, Cat. No.: RT200, Korea). Thereafter, RT-PCR was performed using a PCR kit (enzynomics, Cat. No.: R581T, Korea). The sequences of the primers of Collal, fibronectin, and elastin used in RT-PCR and the sequence of the primer of GAPDH used to compare the total RNA amount of each treatment group are shown in [Table 2].

As a result, as illustrated in FIGS. 5A to 5D, it was confirmed that the mRNA levels of Collal, fibronectin, and elastin decreased by UV irradiation increased again when the NIH3T3 cells were treated with the peptide of the present invention.

From this, it may be appreciated that as the expression levels of the ECM components decreased by UV irradiation in fibroblasts were restored again by the peptide containing the amino acid sequence of SEQ ID NO: 1, the photoaging phenomenon of the cells was inhibited.

### 2-3. Confirmation of Inhibition of Expression of MMP-1 Increased by Ultraviolet Rays

The expression of MMP-1 encoding proteins related to collagen degradation increases by ultraviolet irradiation. Therefore, it was confirmed whether the expression of the MMP-1 protein was inhibited again and the cell aging phenomenon was inhibited when treated with the peptide of the present invention.

First, NIH3T3 cells and HaCaT cells were seeded in a 6-well plate at a cell density of 5 × 10⁵ cell/well, respectively, and then the cells were cultured in DMEM medium containing 10% fetal bovine serum (FBS) for 24 hours. The cultured cells were washed once with serum free DMEM medium, and the peptide of the present invention was mixed in 1 mL of the serum free DMEM medium at each of concentrations of 1, 10, and 50 µM, and dispensed into the cells. A positive control (PC) was treated with 2.5 mM of N-Acetylcysteine (NaC) instead of the above peptide.

The cells were cultured in a CO₂ incubator at 37°C for 1 hour. The medium of the cultured cells was transferred to a tube (e-tube), 1 ml of PBS was added, and then the NIH3T3 cells were irradiated with 6 J/cm² of UVA and the HaCaT cells were irradiated with 15 mJ/cm² of UVB using a UV irradiator (VILBER LOURMAT, Cat No.: 3102-BSU, France). After removing PBS, 900 µL of the medium transferred to the tube was added, and culture was performed in a CO₂ incubator at 37°C for 24 hours. The medium of the cultured cells was transferred to a tube, the cells were washed twice with PBS, and then a cell lysis buffer was added to lyse the cells. A sample was prepared by treating the cells with 5X sample buffer, and then SDS-PAGE was performed using 10% SDS-PAGE gel. The proteins isolated through SDS-PAGE were transferred to a PVDF membrane. Blocking was performed at room temperature for 1 hour using 5% skim milk. Anti-MMP-1 antibodies (Abeam, Cat. No.: ab137332, UK) were diluted to 1:1000 in 5% skim milk and reacted with the membrane for 2 hours. Subsequently, washing was performed three times for 10 minutes each with 0.1% PBS-T (0.1% Tween-20 in PBS). Goat anti-rabbit IgG (Jackson Immune Research, Cat. No.: 111-035-033, USA) was diluted to 1:3000 in 5% skim milk and reacted with the membrane for 2 hours. Thereafter, detection was performed with a film using ECL solution (GE Healthcare, Cat. No.: RPN2232, USA). β-actin was identified using an anti-β-actin antibody (Santacruz biotechnology, USA) to compare the total amount of protein used in the experiment.

As a result, as illustrated in FIGS. 6A and 6B, it was confirmed that the expression of MMP-1 increased by UV irradiation in the NIH3T3 cells and the HaCaT cells was inhibited by the peptide of the present invention.

Through this, it may be appreciated that as the expression of MMP-1 increased by UV irradiation in fibroblasts and keratinocytes decreases again by the peptide containing the amino acid sequence of SEQ ID NO: 1, and the aging phenomenon of the cells is inhibited, and thus the effect of reducing skin wrinkles is obtained.

### 2-4. Confirmation of Increase in Expression of Genes (SIRT1 and AQP3) Related to Skin Barrier Function Decreased by Ultraviolet Rays

The expression of genes related to aging is inhibited when irradiated with UV. Therefore, it was confirmed whether the photoaging phenomenon of the cells was inhibited by confirming whether the expression levels of AQP3 and SIRT1 were restored and increased again when treated with the peptide of the present invention.

First, HaCaT cells were seeded in a 6-well plate at a cell density of 5 × 10⁵ cell/well, and then the cells were cultured in DMEM medium containing 10% fetal bovine serum (FBS) for 24 hours. The cultured cells were washed once with serum free DMEM medium, and then the peptide of the present invention was mixed in 1 mL of the serum free DMEM medium at each of concentrations of 1, 10, and 50 µM, and dispensed into the cells. A positive control (PC) was treated with 2.5 mM of N-Acetylcysteine (NaC) instead of the above peptide.

The cells were cultured in a CO₂ incubator at 37°C for 1 hour. The medium of the cultured cells was transferred to a tube (e-tube), 1 ml of PBS was added, and irradiation was performed with 20 mJ/cm² of UVB using a UV irradiator (VILBER LOURMAT, Cat No.: 3102-BSU, France). After removing PBS, 900 µL of the medium transferred to the tube was added, and culture was performed in a CO₂ incubator at 37°C for 4 hours. The cultured cells were washed twice with PBS, and then RNA was extracted from the cells using easy blue (iNtRON, Cat. No.: 17061, Korea). The extracted RNA was subjected to RT reaction using a kit (enzynomics, Cat. No.: RT200, Korea). Thereafter, RT-PCR was performed using a PCR kit (enzynomics, Cat. No.: R581T, Korea). The sequences of the primers of AQP3 and SIRT1 used in RT-PCR and the sequence of the primer of GAPDH used to compare the total RNA amount of each treatment group are shown in [Table 2].

As a result, as illustrated in FIGS. 7A to 7C, it was confirmed that the mRNA levels of AQP3 and SIRT1 decreased by UV irradiation increased again when treated with the peptide.

From this, it may be appreciated that as the expression levels of AQP3 and SIRT1, which are skin barrier strengthening factors that decrease by UV irradiation, increase again in keratinocytes by the peptide containing the amino acid sequence of SEQ ID NO: 1, the effect of inhibiting or ameliorating aging by ultraviolet rays is obtained.

As described above, although the present invention has been described in detail with reference to only embodiments described herein, it will be apparent to those skilled in the art that various modifications and alterations may be made without departing from the technical idea of the present invention, and it is obvious that these modifications and alterations are within the following claims.

## Claims

1. A peptide comprising an amino acid sequence of SEQ ID NO: 1.

2. The peptide of claim 1, wherein the peptide has skin aging inhibition activity or skin regeneration activity.

3. A composition for skin aging inhibition or skin regeneration, the composition comprising the peptide of claim 1 as an active ingredient.

4. The composition of claim 3, wherein the composition improves resistance to damage to cells caused by aging or an external stimulus.

5. The composition of claim 4, wherein the external stimulus is ultraviolet rays or infrared rays.

6. The composition of claim 4, wherein the cells are skin fibroblasts or keratinocytes.

7. The composition of claim 3, wherein the composition i) increases expression or secretion of collagen, fibronectin, elastin, or hyaluronic acid, and ii) increases expression of SIRT1 or AQP3.

8. The composition of claim 3, wherein the composition i) inhibits production of reactive oxygen species (ROS) caused by ultraviolet rays, ii) increases expression of collagen, fibronectin, or elastin decreased by ultraviolet rays, and iii) inhibits expression of MMP-1 increased by ultraviolet rays.

9. A cosmetic composition for skin aging inhibition or skin regeneration, the cosmetic composition comprising the peptide of claim 1 as an active ingredient.

10. The cosmetic composition of claim 9, wherein the skin aging inhibition or the skin regeneration is prevention or reduction of skin wrinkles, skin elasticity improvement, skin barrier strengthening, prevention of pigmentation, or amelioration of skin photoaging.

11. A pharmaceutical composition for preventing or treating photoaging, the pharmaceutical composition comprising the peptide of claim 1 as an active ingredient.
